# EUROPEAN PATENT SPECIFICATION

(11) **EP 4 237 047 B1**
(45) Date of publication and mention of the grant of the patent: **09.12.2026**
(21) Application number: 21887385.9
(22) Date of filing: 27.10.2021
(51) Int. Cl.: A61M 5/32, A61J 1/05, A61J 1/20, A61M 5/178, A61M 5/34, A61M 39/10

(54) **MEMBRANE WITH GUIDE SURFACE**
MEMBRAN MIT LEITOBERFLÄCHE
MEMBRANE AVEC SURFACE DE GUIDAGE

(30) Priority: 28.10.2020 US 202063106701 P
(43) Date of publication of application: 06.09.2023
(73) Proprietor: Becton, Dickinson and Company, Franklin Lakes, NJ 07417 (US)
(72) Inventor: SHEVGOOR, Siddarth, K., Mission Viejo, California 92692 (US); SADASIVAM, Balaji, Mahwah, New Jersey 07430 (US); DISTEFANO, Nicole, Jean, Dumont, New Jersey 07628 (US)
(74) Representative: dompatent
(86) International application number: PCT/US2021/056726
(87) International publication number: WO 2022/093878

(56) References cited:
- WO-A1-2012/117648
- US-A1- 2013 076 019
- US-A1- 2014 261 877
- US-A1- 2018 200 148
- US-A1- 2019 290 543
- US-B2- 9 999 569

## Description

### CROSS-REFERENCE TO RELATED APPLICATION

The present application claims priority to United States Provisional Application Serial No. 63/106,701, filed October 28, 2020, entitled "Membrane with Guide Surface".

### BACKGROUND OF THE INVENTION

### Field of the Disclosure

The present disclosure relates generally to a membrane for a closed system transfer device.

### Description of the Related Art

Health care providers reconstituting, transporting, and administering hazardous drugs, such as cancer treatments, can put health care providers at risk of exposure to these medications and present a hazard in the health care environment. Unintentional chemotherapy exposure can affect the nervous system, impair the reproductive system, and bring an increased risk of developing blood cancers in the future. Some drugs must be dissolved or diluted before they are administered, which involves transferring a solvent from one container to a sealed vial containing the drug in powder or liquid form, by means of a needle. Drugs may be inadvertently released into the atmosphere in gas form or by way of aerosolization, during the withdrawal of the needle from the vial and while the needle is inside the vial if any pressure differential between the interior of the vial and the surrounding atmosphere exists. In order to reduce the risk of health care providers being exposed to toxic drugs, the transfer of these drugs is accomplished utilizing a closed system transfer device or system.

Closed system transfer devices or systems may utilize membranes to ensure the safe transfer of fluid between components. For example, a syringe adapter may include a membrane that contacts a membrane of a mating component, such as a patient connector, IV bag spike, or vial adapter.

A membrane for a closed system transfer device having the features defined within the preamble of claim 1 is described in US2013/0076019.

### SUMMARY OF THE INVENTION

A membrane for a closed system transfer device according to the features defined within claim 1 includes a body having a first end and a second end positioned opposite the first end, the body defining a passageway extending from the first end of the body to a position intermediate the first end of the body and the second end of the body, and a guide member positioned within the passageway of the body. The guide member having a first end and a second end positioned opposite the first end, with the guide member having a guide surface configured to engage a needle positioned within the passageway of the body.

The guide member may be formed integrally with the body. The guide member may be formed as a projection extending from the body in a radially inward direction. The guide member may be a plurality of equally-spaced projections extending from the body in a radially inward direction. The guide surface may be concave. The guide member may be positioned intermediate the first and second ends of the body. The guide member may extend at least 25 percent of the length of the body. The body may define a slit extending from the passageway to the second end of the body. The passageway may include a tapered terminal end. The guide surface may be convex.

In a further aspect or embodiment, a syringe adapter includes a housing having a connector configured to be secured to a syringe, a needle received within the housing and in fluid communication with the connector, with the needle having a first end and a second end positioned opposite the first end, and the membrane of any of the aspects or embodiments discussed above. The membrane is moveable from a first position within the housing where the second end of the needle is received within the passageway of the membrane to a second position where the second end of the needle is positioned outside of the passageway of the membrane. The needle is engaged with the guide member when the membrane moves from the first position to the second position.

The syringe adapter may further include a collet defining an opening, with the membrane received within the opening of the collet. An outer diameter of the needle may taper from the first end of the needle to the second end of the needle. The tapered terminal end of the passageway of the membrane may correspond to the shape of the second end of the needle.

### BRIEF DESCRIPTION OF THE DRAWINGS

The above-mentioned and other features and advantages of this disclosure, and the manner of attaining them, will become more apparent and the disclosure itself will be better understood by reference to the following descriptions of aspects of the disclosure taken in conjunction with the accompanying drawings, wherein:
FIG. 1 is a top perspective view of a membrane according to one aspect or embodiment of the present application.
FIG. 2 is a bottom perspective view of the membrane of FIG. 1.
FIG. 3 is a front view of the membrane of FIG. 1.
FIG. 4 is a cross-sectional view of the membrane taken along line A-A in FIG. 3 according to one aspect or embodiment of the present application.
FIG. 5 is an enlarged cross-sectional view of the area shown in FIG. 4.
FIG. 6 is a cross-sectional view of the membrane taken along line A-A according to a second aspect or embodiment of the present application.
FIG. 7 is a cross-sectional view of the membrane taken along line A-A according to a third aspect or embodiment of the present application.
FIG. 8 is an enlarged cross-sectional view of the area shown in FIG. 7.
FIG. 9 is a cross-sectional view of the membrane taken along line A-A according to a fourth aspect or embodiment of the present application
FIG. 10 is an enlarged cross-sectional view of the area shown in FIG. 9.
FIG. 11 is a perspective view of a system according to one aspect or embodiment of the present application.
FIG. 12 is a cross-sectional view of the system of FIG. 11.
FIG. 13 is a cross-sectional view of the system of FIG. 11, showing a syringe adapter connected to a patient connector.
FIG. 14 is a perspective view of a needle according to one aspect or embodiment of the present application.
FIG. 15 is a side view of the needle of FIG. 14.

Corresponding reference characters indicate corresponding parts throughout the several views. The exemplifications set out herein illustrate exemplary aspects of the disclosure, and such exemplifications are not to be construed as limiting the scope of the disclosure in any manner.

### DETAILED DESCRIPTION

The following description is provided to enable those skilled in the art to make and use the described aspects contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary aspects of the invention. Hence, specific dimensions and other physical characteristics related to the aspects disclosed herein are not to be considered as limiting.

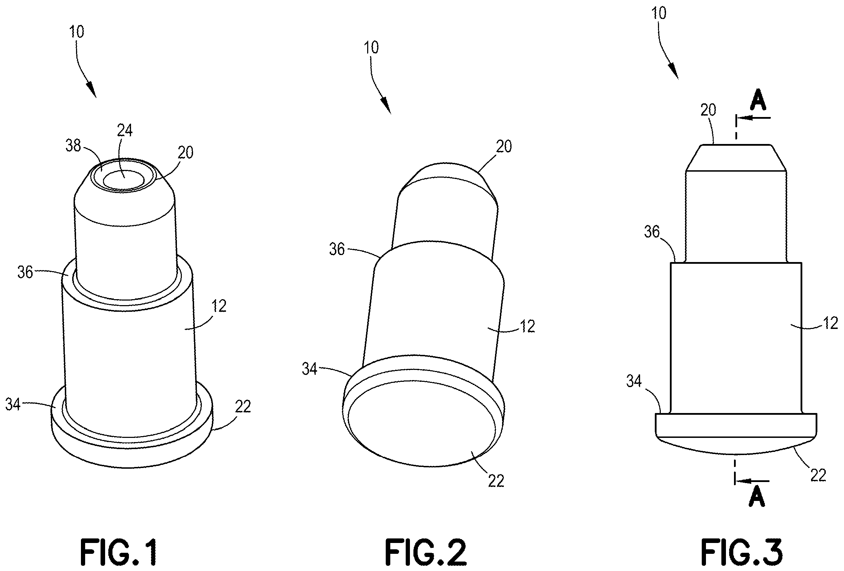

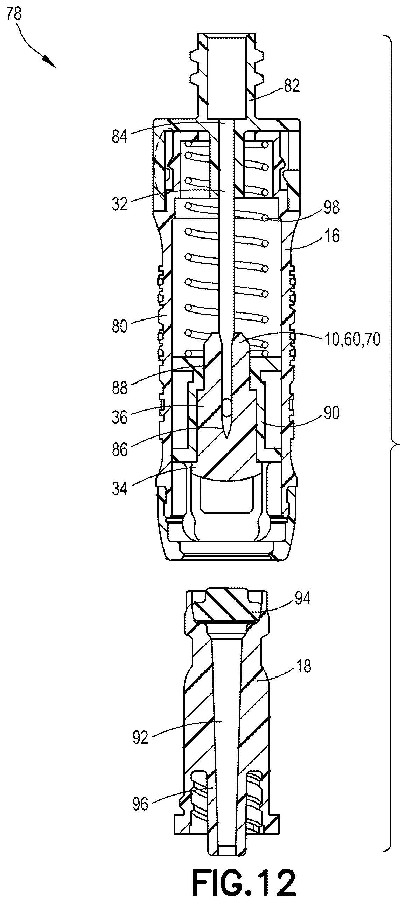

Referring to FIGS. 1-5 and 11-13, a membrane 10 for a closed system transfer device according to one aspect or embodiment of the present application includes a body 12 and a guide member 14. As shown in FIG. 12, the membrane 10 is shown in connection with a syringe adapter 16, which is utilized to connect a syringe barrel (not shown) to another component of a closed system transfer device or system, such as a patient connector 18, vial adapter, IV bag spike, etc. For example, the syringe adapter 16 may be used to facilitate the transfer of fluid from one container, such as a syringe barrel, to another container or line, such as an intravenous line, IV bag, or other component. The membrane 10 may be utilized in any component of a closed system transfer device or system. The syringe adapter 16 may be the same and operate in the same manner as the syringe adapter shown and described in United States Patent Application Publication No. 2015/0297454, which is hereby incorporated by reference in its entirety.

Referring to FIGS. 1-5, the body 12 has a first end 20 and a second end 22 positioned opposite the first end 20, with the body 12 defining a passageway 24 extending from the first end 20 of the body 12 to a position intermediate the first end 20 of the body 12 and the second end 22 of the body 12. The guide member 14 is positioned within the passageway 24 of the body 12, with the guide member 14 having a first end 26 and a second end 28 positioned opposite the first end 26. The guide member 14 has a guide surface 30 configured to engage a needle 32 positioned within the passageway 24 of the body 12. In one aspect or embodiment, the guide surface 30 of the guide member 14 is configured to guide movement of the needle 32 passing through the passageway 24 and through the membrane 10 to ensure the needle 32 passes through the same location during use of the syringe adapter 16.

Referring to FIGS. 1-4, the second end 22 of the membrane 10 includes a convex surface and a first flange 34 extending radially outward from the body 12. The convex surface is configured to engage a membrane of a mating component, such as the patient connector 18, as discussed in more detail below. The membrane 10 also includes a second flange 36 positioned intermediate the first and second ends 20, 22 of the membrane 10. The first end 20 of the membrane 10 is tapered radially inward. The passageway 24 includes a conical portion 38 at the first end 20 of the membrane 10, a first cylindrical portion 40 extending from the conical portion 38, a second cylindrical portion 42 extending from the first cylindrical portion 40, and a tapered terminal end 44 extending from the second cylindrical portion 42. The first cylindrical portion 40 is narrower than the second cylindrical portion 42. The body 12 is formed from an elastomeric material, although other suitable materials may be utilized.

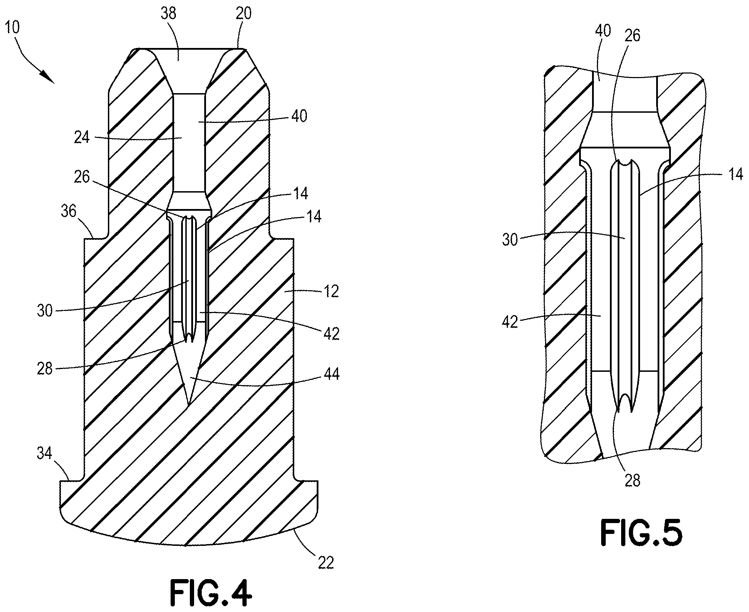

Referring to FIGS. 4 and 5, in one aspect or embodiment, the guide member 14 is formed integrally with the body 12, although the guide member 14 may be formed separately and secured to the body 12. The guide member 14 is a projection extending from the body 12 in a radially inward direction. As shown in FIG. 5, the guide member 14 is formed as a plurality of equally-spaced projections extending from the body 12 in a radially inward direction, although one or more projections may be provided. The guide surface 30 of the guide member 14 is concave and configured to receive an outside surface 46 of the needle 32 of the syringe adapter 16. The guide member 14 is positioned intermediate the first and second ends 20, 22 of the body 12. More specifically, the guide member 14 is positioned within the second cylindrical portion 42 of the passageway 24. In one aspect or embodiment, the guide member 14 extends at least 25% of the length of the body 12, although the guide member 14 may extend at least 10%, 15%, 20%, 30%, 35%, 40%, 45%, or 50% of the length of the body 12.

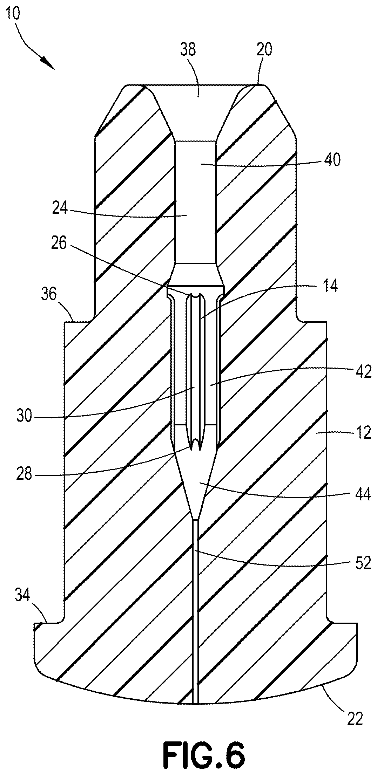

Referring to FIG. 6, in one aspect or embodiment, the body 12 of the membrane 10 defines a slit 52 extending from the tapered terminal end 44 to the second end 22 of the body 12. The slit 52 is positioned co-axially with the passageway 24, with the slit 52 configured to receive the needle 32 of the syringe adapter 16 during use of the syringe adapter 16. In one aspect or embodiment, the slit 52 is smaller than an outer diameter of the needle 32.

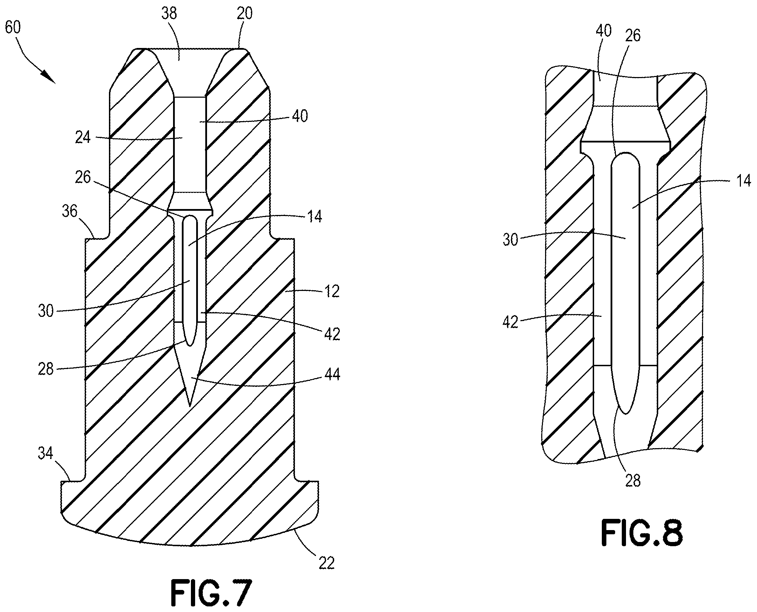

Referring to FIGS. 7 and 8, a membrane 60 for a closed system transfer device according to a second aspect or embodiment of the present application is shown. The membrane 60 is similar to the membrane 10 shown in FIGS. 1-5 and discussed above, except the guide surface 30 of the guide member 14 is convex. The membrane 10 functions in the same manner as the membrane 60 of FIGS. 1-5 with the convex shape of the guide surface 30 configured to engage and guide the needle 32 of the syringe adapter 16. In one aspect or embodiment, the convex shape of the guide surface 30 of the guide member 14 has less surface area contact with the needle 32 compared to the guide surface 30 shown in FIGS. 4 and 5.

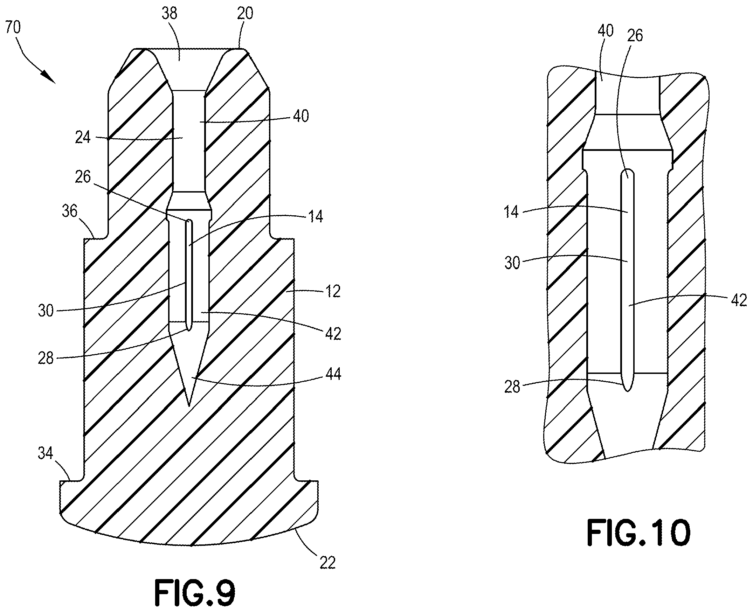

Referring to FIGS. 9 and 10, a membrane 70 for a closed system transfer device according to a third aspect or embodiment of the present application is shown. The membrane 70 is similar to the membrane 60 shown in FIGS. 7 and 8, except the guide member 14 is narrower than the guide member 14 of FIGS. 7 and 8. The size, shape, and configuration of the guide member 14 may be optimized to ensure the needle 32 of the syringe adapter 16 is guided through the same spot of the membrane 10, 60, 70 during use of the syringe adapter 16. In one aspect or embodiment, the guide member 14 of FIGS. 9 and 10 applies less compression to the needle 32 compared to the guide member 14 of FIGS. 7 and 8, but reduces friction between the guide member 14 and the needle 32 thereby reducing the force required to move the needle 32 through the membrane 70.

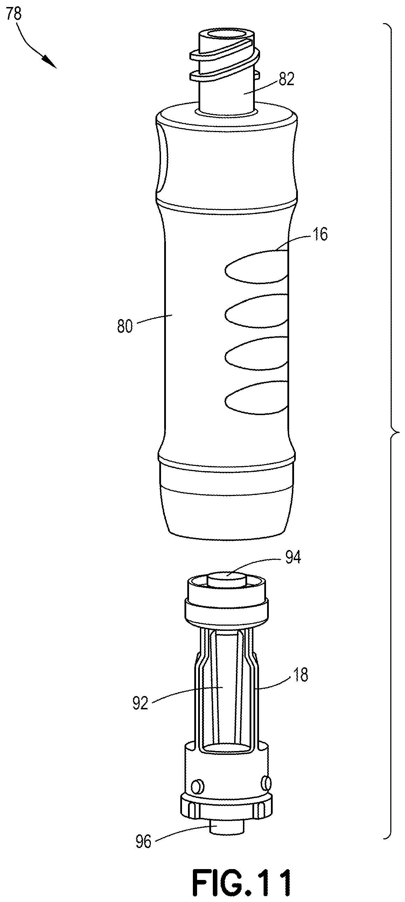

Referring to FIGS. 11-13, a system according to one aspect or embodiment includes the syringe adapter and the patient connector 18 or other suitable mating connector, such as an IV bag spike, vial adapter, etc. The syringe adapter 16 includes a housing 80 having a connector 82 configured to be secured to a syringe, the needle 32 received within the housing 80 and in fluid communication with the connector 82, with the needle 32 having a first end 84 and a second end 86 positioned opposite the first end 84, and the membrane 10, 60, 70 as shown in any of FIGS. 1-8. The membrane 10, 60, 70 is moveable from a first position within the housing 80 where the second end 86 of the needle 32 is received within the passageway 24 of the membrane 10, 60, 70 to a second position where the second end 86 of the needle 32 is positioned outside of the passageway 24 of the membrane 10, 60, 70. The needle 32 is engaged with the guide member 14 when the membrane 10, 60, 70 moves from the first position to the second position. The patient connector 18 includes a body 92, a membrane 94, and a connector 96. The connector 96 is male luer connector, although other suitable connectors may be utilized.

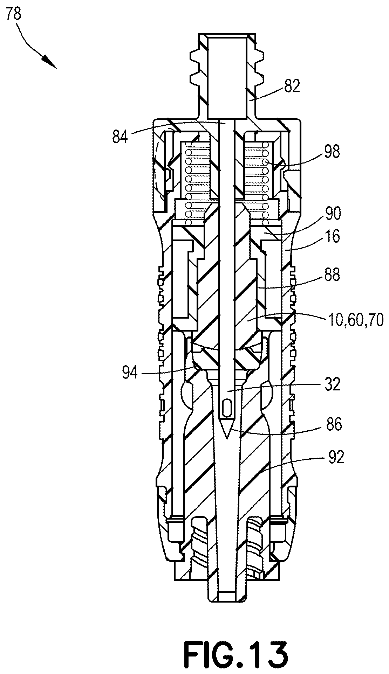

In one aspect or embodiment, the membrane 10, 60, 70 is received by an opening 88 defined by a collet 90. The first flange 34 and second flange 36 of the membrane 10, 60, 70 correspond to the opening 88 of the collet 90 and are configured to secure the membrane 10, 60, 70 within the collet 90. The collet 90 and the membrane 10, 60, 70 are moveable between the first and second positions when a mating connector, such as the patient connector 18, is received by the housing 80 and the collet 90 to move the collet 90 and the membrane 10, 60, 70 from the first position (shown in FIG. 12) to the second position (shown in FIG. 13) such that the needle 32 is placed in fluid communication with the patient connector 18. As shown in FIG. 13, the membrane 94 of the patient connector 18 engages the membrane 10, 60, 70 of the syringe adapter 16, which ensures a drip-free closed connections as the needle 32 passes through the membrane 10, 60, 70 of the syringe adapter and the membrane 94 of the patient connector 18. The collet 90 is configured to engage a portion of the body 92 of the patient connector 18 to secure the collet 90 to the patient connector 18 when the collet 90 moves from the first position to the second position. The syringe adapter 16 may include a spring 98 to bias the collet 90 and the membrane 10, 60, 70 to the first position. The tapered terminal end 44 of the passageway 24 of the membrane 10, 60, 70 corresponds to the shape of the second end 86 of the needle 32, which facilitates, along with the guide member 14, the movement of the needle 32 of the syringe adapter 16 through the same spot of the membrane 10, 60, 70. The operation of the syringe adapter 16 is described in United States Patent Application Publication No. 2015/0297454.

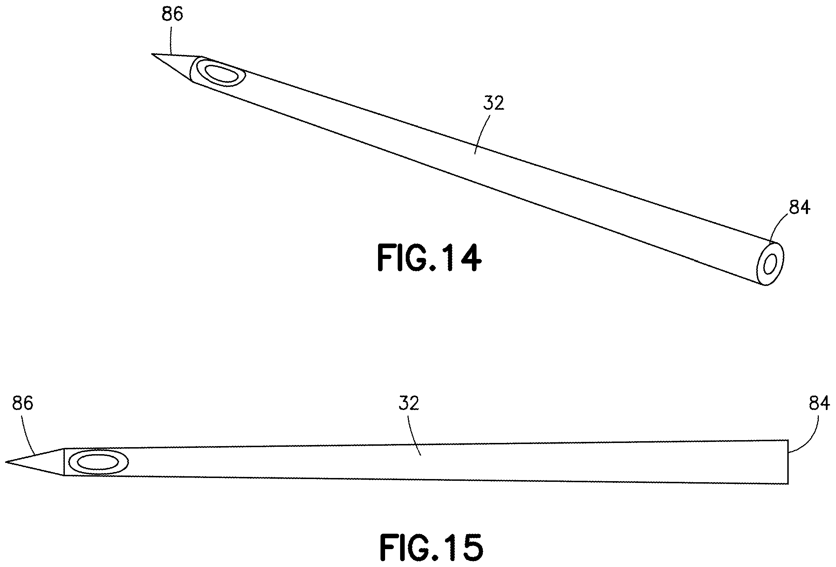

Referring to FIGS. 14 and 15, in one aspect or embodiment of the present application, an outer diameter of the needle 32 tapers from the first end 84 of the needle 32 to the second end 86 of the needle 32. When the membrane 10, 60, 70 moves from the first position to the second position, the larger diameter portion of the needle 32 passes by the guide member 14 to provide a needle feeding effect to further ensure a consistent trajectory path of the needle 32 during use of the syringe adapter 16. The tapering of the needle 32 provides a taper lock with the tapered internal geometry of the membrane 10, 60, 70. Further, the taper of the needle 32 shown in FIGS. 14 and 15 allows for smaller frictional forces to be overcome, while still providing diametrical compression to aid in centering of the needle 32. A tapered needle also minimizes coring and maximizes the self-sealing capability of the membrane. The self-sealing capability of the membrane helps minimize vapor and/or liquid leakage onto the surface.

While this disclosure has been described as having exemplary designs, the present disclosure can be further modified within the scope of this disclosure. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims. To the extent possible, one or more features of any aspect or embodiment discussed above can be combined with one or more features of any other aspect or embodiment.

## Claims

1. A membrane (10) for a closed system transfer device comprising:
a body (12) having a first end (20) and a second end (22) positioned opposite the first end (20), the body (12) defining a passageway (24) extending from the first end (20) of the body (12) to a position intermediate the first end (20) of the body (12) and the second end (22) of the body (12); and
a guide member (14) positioned within the passageway (24) of the body (12), the guide member (14) having a first end (26) and a second end (28) positioned opposite the first end (26), the guide member (14) having a guide surface (30) configured to engage a needle (32) positioned within the passageway (24) of the body (12);
**characterized in that**
the guide member (14) comprises a plurality of equally spaced projections extending from the body (12) in a radially inward direction.

2. The membrane (10) of claim 1, wherein the guide member (14) is formed integrally with the body (12).

3. The membrane (10) of any of claims 1-2, wherein the guide surface (30) is concave.

4. The membrane (10) of any of claims 1-3, wherein the guide member (14) is positioned intermediate the first and second ends (22) of the body (12).

5. The membrane (10) of any of claims 1-4, wherein the guide member (14) extends at least 25 percent of the length of the body (12).

6. The membrane (10) of any of claims 1-5, wherein the body (12) defines a slit (52) extending from the passageway (24) to the second end (22) of the body (12).

7. The membrane (10) of any of claims 1-6, wherein the passageway (24) comprises a tapered terminal end (44).

8. The membrane (10) of any of claims 1-7, wherein the guide surface (30) is convex.

9. A syringe adapter (16) comprising:
a housing (80) having a connector (82) configured to be secured to a syringe;
a needle (32) received within the housing (80) and in fluid communication with the connector (82), the needle (32) having a first end (84) and a second end (86) positioned opposite the first end (84); and
the membrane (10) of any of claims 1-8, the membrane moveable from a first position within the housing (80) where the second end (86) of the needle (32) is received within the passageway (24) of the membrane (10) to a second position where the second end (86) of the needle (32) is positioned outside of the passageway (24) of the membrane (10), the needle (32) is engaged with the guide member (14) when the membrane (10) moves from the first position to the second position.

10. The syringe adapter (16) of claim 9, further comprising a collet (90) defining an opening (88), the membrane (10) received within the opening (88) of the collet (90).

11. The syringe adapter (16) of claim 9 or claim 10, wherein an outer diameter of the needle tapers from the first end (84) of the needle (32) to the second end (86) of the needle (32).

12. The syringe adapter (16) of any of claims 9-11, wherein the tapered terminal end of the passageway (24) of the membrane corresponds to the shape of the second end (836) of the needle (32).

## Patentansprüche

1. Membran (10) für eine Transfervorrichtung mit geschlossenem System, umfassend: einen Körper (12), der ein erstes Ende (20) und ein zweites Ende (22) aufweist, das gegenüber dem ersten Ende (20) angeordnet ist, wobei der Körper (12) einen Kanal (24) definiert, der sich von dem ersten Ende (20) des Körpers (12) bis zu einer Position zwischen dem ersten Ende (20) des Körpers (12) und dem zweiten Ende (22) des Körpers (12) erstreckt; und ein Leitelement (14), das in dem Kanal (24) des Körpers (12) angeordnet ist, wobei das Leitelement (14) ein erstes Ende (26) und ein zweites Ende (28) aufweist, das gegenüber dem ersten Ende (26) angeordnet ist, wobei das Leitelement (14) eine Leitoberfläche (30) aufweist, die dazu ausgebildet ist, mit einer Nadel (32) in Eingriff zu treten, die in dem Kanal (24) des Körpers (12) angeordnet ist; **dadurch gekennzeichnet, dass** das Leitelement (14) mehrere gleichmäßig beabstandete Vorsprünge umfasst, die sich von dem Körper (12) in einer radial nach innen weisenden Richtung erstrecken.

2. Membran (10) nach Anspruch 1, wobei das Leitelement (14) einstückig mit dem Körper (12) ausgebildet ist.

3. Membran (10) nach einem der Ansprüche 1 bis 2, wobei die Leitoberfläche (30) konkav ist.

4. Membran (10) nach einem der Ansprüche 1 bis 3, wobei das Leitelement (14) zwischen dem ersten und dem zweiten Ende (22) des Körpers (12) angeordnet ist.

5. Membran (10) nach einem der Ansprüche 1 bis 4, wobei sich das Leitelement (14) über mindestens 25 Prozent der Länge des Körpers (12) erstreckt.

6. Membran (10) nach einem der Ansprüche 1 bis 5, wobei der Körper (12) einen Schlitz (52) definiert, der sich von dem Kanal (24) bis zu dem zweiten Ende (22) des Körpers (12) erstreckt.

7. Membran (10) nach einem der Ansprüche 1 bis 6, wobei der Kanal (24) ein sich verjüngendes terminales Ende (44) umfasst.

8. Membran (10) nach einem der Ansprüche 1 bis 7, wobei die Leitoberfläche (30) konvex ist.

9. Spritzenadapter (16), umfassend: ein Gehäuse (80), das einen Konnektor (82) aufweist, der dazu ausgebildet ist, an einer Spritze befestigt zu werden; eine Nadel (32), die in dem Gehäuse (80) aufgenommen ist und in Fluidverbindung mit dem Konnektor (82) steht, wobei die Nadel (32) ein erstes Ende (84) und ein zweites Ende (86) aufweist, das gegenüber dem ersten Ende (84) angeordnet ist; und die Membran (10) nach einem der Ansprüche 1 bis 8, wobei die Membran von einer ersten Position in dem Gehäuse (80), in der das zweite Ende (86) der Nadel (32) in dem Kanal (24) der Membran (10) aufgenommen ist, in eine zweite Position bewegbar ist, in der das zweite Ende (86) der Nadel (32) außerhalb des Kanals (24) der Membran (10) angeordnet ist, wobei die Nadel (32) mit dem Leitelement (14) in Eingriff steht, wenn sich die Membran (10) von der ersten Position in die zweite Position bewegt.

10. Spritzenadapter (16) nach Anspruch 9, ferner umfassend eine Spannhülse (90), die eine Öffnung (88) definiert, wobei die Membran (10) in der Öffnung (88) der Spannhülse (90) aufgenommen ist.

11. Spritzenadapter (16) nach Anspruch 9 oder Anspruch 10, wobei sich ein Außendurchmesser der Nadel von dem ersten Ende (84) der Nadel (32) bis zu dem zweiten Ende (86) der Nadel (32) verjüngt.

12. Spritzenadapter (16) nach einem der Ansprüche 9 bis 11, wobei das sich verjüngende terminale Ende des Kanals (24) der Membran der Form des zweiten Endes (836) der Nadel (32) entspricht.

## Revendications

1. Membrane (10) pour un dispositif de transfert à système fermé comprenant :
un corps (12) présentant une première extrémité (20) et une seconde extrémité (22) positionnée à l'opposé de la première extrémité (20), le corps (12) définissant un passage (24) s'étendant de la première extrémité (20) du corps (12) à une position intermédiaire entre la première extrémité (20) du corps (12) et la seconde extrémité (22) du corps (12) ; et
un organe de guidage (14) positionné au sein du passage (24) du corps (12), l'organe de guidage (14) présentant une première extrémité (26) et une seconde extrémité (28) positionnée à l'opposé de la première extrémité (26), l'organe de guidage (14) présentant une surface de guidage (30) configurée pour se mettre en prise avec une aiguille (32) positionnée au sein du passage (24) du corps (12) ;
**caractérisée en ce que**
l'organe de guidage (14) comprend une pluralité de saillies équidistantes s'étendant à partir du corps (12) dans une direction radialement vers l'intérieur.

2. Membrane (10) selon la revendication 1, dans laquelle l'organe de guidage (14) est formé solidairement avec le corps (12).

3. Membrane (10) selon l'une quelconque des revendications 1 et 2, dans laquelle la surface de guidage (30) est concave.

4. Membrane (10) selon l'une quelconque des revendications 1 à 3, dans laquelle l'organe de guidage (14) est à une position intermédiaire entre les première et seconde extrémités (22) du corps (12).

5. Membrane (10) selon l'une quelconque des revendications 1 à 4, dans laquelle l'organe de guidage (14) s'étend d'au moins 25 % de la longueur du corps (12).

6. Membrane (10) selon l'une quelconque des revendications 1 à 5, dans laquelle le corps (12) définit une fente (52) s'étendant du passage (24) à la seconde extrémité (22) du corps (12).

7. Membrane (10) selon l'une quelconque des revendications 1 à 6, dans laquelle le passage (24) comprend une extrémité terminale effilée (44).

8. Membrane (10) selon l'une quelconque des revendications 1 à 7, dans laquelle la surface de guidage (30) est convexe.

9. Adaptateur de seringue (16) comprenant :
un boîtier (80) présentant un connecteur (82) configuré pour être fixé à une seringue ;
une aiguille (32) reçue au sein du boîtier (80) et en communication fluidique avec le connecteur (82), l'aiguille (32) présentant une première extrémité (84) et une seconde extrémité (86) positionnée à l'opposé de la première extrémité (84) ; et
la membrane (10) selon l'une quelconque des revendications 1 à 8, la membrane pouvant se déplacer d'une première position au sein du boîtier (80) où la seconde extrémité (86) de l'aiguille (32) est reçue au sein du passage (24) de la membrane (10) vers une seconde position où la seconde extrémité (86) de l'aiguille (32) est positionnée à l'extérieur du passage (24) de la membrane (10), l'aiguille (32) étant mise en prise avec l'organe de guidage (14) lorsque la membrane (10) se déplace de la première position à la seconde position.

10. Adaptateur de seringue (16) selon la revendication 9, comprenant en outre une virole (90) définissant une ouverture (88), la membrane (10) étant reçue au sein de l'ouverture (88) de la virole (90).

11. Adaptateur de seringue (16) selon la revendication 9 ou la revendication 10, dans lequel un diamètre extérieur de l'aiguille s'effile de la première extrémité (84) de l'aiguille (32) à la seconde extrémité (86) de l'aiguille (32).

12. Adaptateur de seringue (16) selon l'une quelconque des revendications 9 à 11, dans lequel l'extrémité terminale effilée du passage (24) de la membrane correspond à la forme de la seconde extrémité (836) de l'aiguille (32).
